# NEUE EUROPÄISCHE PATENTSCHRIFT

(11) **EP 2 280 767 B2**
(45) Veröffentlichungstag und Bekanntmachung des Hinweises auf die Entscheidung über den Einspruch: **05.01.2022**
(45) Hinweis auf die Patenterteilung: 11.06.2014
(21) Anmeldenummer: 09734866.8
(22) Anmeldetag: 04.04.2009
(51) Int. Cl.: A61Q 17/04, A61K 8/49, A61K 8/41

(54) **LICHTSCHUTZFILTERKOMBINATION MIT 2,4,6-TRIS-(BIPHENYL)-1,3,5-TRIAZIN**
LIGHT PROTECTION FILTER COMBINATION COMPRISING 2,4,6-TRIS-(BIPHENYL)-1,3,5-TRIAZINE
COMBINAISON DE FILTRE DE PROTECTION SOLAIRE CONTENANT 2,4,6-TRIS-(BIPHÉNYL)-1,3,5-TRIAZINE

(30) Priorität: 25.04.2008 DE 102008021631
(43) Veröffentlichungstag der Anmeldung: 09.02.2011
(73) Patentinhaber: Beiersdorf AG, 20253 Hamburg (DE)
(72) Erfinder: SKUBSCH, Kerstin, 25497 Prisdorf (DE); BLOHM, Alexandra, 20257 Hamburg (DE); JUNGE, Janina, 20249 Hamburg (DE)
(74) Vertreter: Beiersdorf AG
(86) Internationale Anmeldenummer: PCT/EP2009/002496
(87) Internationale Veröffentlichungsnummer: WO 2009/129924

(56) Entgegenhaltungen:
- EP-A- 1 046 391
- WO-A-2007/012611
- WO-A-2007/072008
- WO-A-2007/075747
- WO-A2-2004/085412
- DE-A1-102004 047 288
- US-A1- 2004 191 191
- ANONYMOUS: "Sunscreen formulations containing a 50% dispersion of micronized Trisbiphenyltriazine" IP.COM JOURNAL, IP.COM INC., WEST HENRIETTA, NY, US, 30. November 2004 (2004-11-30), XP013022367 ISSN: 1533-0001

## Beschreibung

Die vorliegende Erfindung betrifft eine kosmetische Zubereitung enthaltend eine Lichtschutzfilterkombination mit 2,4,6-Tris-(biphenyl)-1,3,5-triazin.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie der Anlage 7 der deutschen Kosmetikverordnung zusammengefasst.

Eine besondere Form von UV-Lichtschutzfiltersubstanzen stellen die Mikropigmente dar. Die UV-Schutzwirkung der Mikropigmente beruht auf den physikalischen Effekten der Reflexion und Lichtstreuung. In kosmetischen Zubereitungen werden als Mikropigmente fast ausschließlich anorganische Mikropigmente aus Titandioxid, Zinkoxid oder Mischoxiden mit zum Beispiel Eisenoxiden eingesetzt.

Die Vorteile von Mikropigmenten als UV-Filtersubstanz in kosmetischen Zubereitungen liegen vor allem darin begründet, dass die Pigmente im Gegensatz zu gelöst oder flüssig vorliegenden, nicht in die Haut penetrieren. Das Auftreten von allergischen Reaktionen ist damit ausgeschlossen.

Nachteilig am Stande der Technik ist jedoch der Umstand, dass Mikropigmente nur schwer stabil in kosmetische Zubereitungen einzuarbeiten sind. Insbesondere wenn Mikropigmente in höheren Konzentrationen (Konzentrationen über 7 Gewichts-%, bezogen auf das Gesamtgewicht der Zubereitung) eingesetzt werden, bilden sie relativ schnell PigmentAgglomerate, die aus der Zubereitung ausfallen. Die Lagerstabilität ist also gering.

In jüngerer Zeit geht der Trend bei kosmetischen Produkten, die u.a. dem UV-Schutz dienen (z.B. Sonnenschutzmittel, Tagespflegecremes) zu hohen Lichtschutzfaktoren bei gleichzeitigem hohem UV-A-Schutz. Bei den "klassischen" anorganischen Filterpigmenten aus Titandioxid und Zinkoxid führen höhere Einsatzkonzentrationen jedoch neben den Problemen bei der Einarbeitung und Stabilität zusätzlich dazu, dass die sensorischen Eigenschaften der Zubereitungen sich deutlich verschlechtern. Insbesondere beim Verteilen der Zubereitungen entsteht ein unangenehmes Hautgefühl. Um den Anforderungen nach hohem Lichtschutzfaktor und hohem UV-A-Schutz gerecht zu werden, kann bisher in der Regel jedoch nicht auf höhere Konzentrationen an anorganischen Filterpigmenten verzichtet werden.

Viele der UVA- und Breitbandfilter führen zu einer deutlichen Gelbfärbung der kosmetischen Zubereitungen, insbesondere, wenn sie in größeren Mengen eingesetzt werden. Besonders deutlich gefärbt sind Formeln, die 2-(4'-Diethylamino-2'-hydoxybenzoyl)-benzoesäurehexylester enthalten. Eine solche Gelbfärbung wird von vielen Verbrauchern als optisch unattraktiv empfunden und ist in der Regel nicht gewünscht.

Es war daher die Aufgabe der vorliegenden Erfindung, die Nachteile des Standes der Technik zu beseitigen. Es sollten Zubereitungen entwickelt werden, die besonders hautfreundlich sind, angenehme sensorische Eigenschaften aufzeigen und einfach und kostengünstig herstellbar sind. Die Zubereitungen sollten dabei möglichst wenig anorganische Metalloxid-Pigmente enthalten und wenig gelb gefärbt sein. Insbesondere sollten die Zubereitungen einen, über den gesamten für den UV-Schutz der Haut relevanten Bereich, gleichmäßigen und ausgewogenen Schutz vor der UV-Strahlung bieten.

Überraschend gelöst werden die Aufgaben durch eine kosmetische Zubereitung gemäß einem der Ansprüche 1 bis 3.

Zwar kennt der Fachmann die DE 10 2004 047288, doch konnte diese Schrift nicht den Weg zur vorliegenden Erfindung weisen.

Überraschenderweise kommt es bei der erfindungsgemäßen Zusammensetzung zu einer überadditiven Steigerung des SPF.

Es ist erfindungsgemäß bevorzugt, wenn die erfindungsgemäße Zubereitung frei ist von Zinkoxid.

Es ist ferner erfindungsgemäß bevorzugt, wenn die erfindungsgemäße Zubereitung frei ist von Titandioxid.

Nicht zuletzt ist es erfindungsgemäß bevorzugt, wenn die erfindungsgemäße Zubereitung frei ist von weiteren UV-Filtern. Dies ist insbesondere vorteilhaft, wenn Zubereitungen mit einem Lichtschutzfaktor bis SPF 30 hergestellt werden sollen.

Es war insbesondere überraschend für den Fachmann, dass die erfindungsgemäßen Zubereitungen ohne Titandioxid einen höheren Lichtschutzfaktor (SPF) aufwiesen, als äquivalente Zubereitungen mit Titandioxid (Zinkoxid).

Erfindungsgemäß vorteilhaft beträgt die Konzentration von 2,4,6-Tris-(biphenyl)-1,3,5-triazin in der Zubereitung von 0,01 bis 15 Gewichts-%, bezogen auf das Gesamtgewicht der Zubereitung.

Erfindungsgemäß bevorzugt beträgt die Konzentration von 2,4,6-Tris-(biphenyl)-1,3,5-triazin in der Zubereitung von 0,1 bis 5 Gewichts-%, bezogen auf das Gesamtgewicht der Zubereitung.

Erfindungsgemäß vorteilhaft beträgt die Konzentration von Phenylbenzimidazolsulfonsäure und deren Salze in der Zubereitung von 0,1 bis 8Gewichts-%, bezogen auf das Gesamtgewicht der Zubereitung.

Erfindungsgemäß bevorzugt beträgt die Konzentration von Phenylbenzimidazolsulfonsäure und deren Salze in der Zubereitung von 0,2 bis 4 Gewichts-%, bezogen auf das Gesamtgewicht der Zubereitung.

Erfindungsgemäß vorteilhaft beträgt die Konzentration von 2-(4'-Diethylamino-2'-hydoxybenzoyl)-benzoesäurehexylester in der Zubereitung von 0,1 bis 10 Gewichts-%, bezogen auf das Gesamtgewicht der Zubereitung.

Erfindungsgemäß bevorzugt beträgt die Konzentration von 2-(4'-Diethylamino-2'-hydoxybenzoyl)-benzoesäurehexylester in der Zubereitung von 0,2 bis 8 Gewichts-%, bezogen auf das Gesamtgewicht der Zubereitung.

Es ist erfindungsgemäß vorteilhaft, wenn die Zubereitung nach Anspruch 1 oder 3 einen oder mehrere UV-Filter gewählt aus der Gruppe der Verbindungen Ethylhexylsalicylat, Homosalat, Benzophenon-3 enthält.

Erfindungsgemäß vorteilhafte Ausführungsformen sind auch dadurch gekennzeichnet, dass die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Terephthalidendicamphersulfonsäure; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,2'-Methylen-bis-(6-(2H-benzotriazol-2-yl)-4-(1,1,3,3-tetramethylbutyl)-phenol); 2,4-Bis-(4'- Di-neopentylaminobenzalmalonat)-6-(4"-butylaminobenzoat)-s-triazin; Polysilicone-15; Merocyanine.

Es ist erfindungsgemäß vorteilhaft, wenn die Zubereitung nach Anspruch 1 oder 2 eine oder mehrere der folgenden lipophilen Komponenten enthält: C12-15Alkylbenzoat, C12-13Alkyltartrat, Dicaprylylether, Octyldodecanol, Cyclomethicon, Dimethicon, Isohexadecan, Myristylmyristat.

Selbstverständlich kann die Zubereitung auch weitere lipophile Bestandteile, Ölkomponenten, Wachse etc. enthalten.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer) sowie ein Copolymer aus Vinylpyrrolidon und Acrylsäure.

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 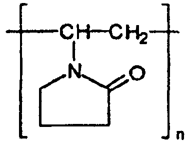 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Obwohl die erfindungsgemäße Zubereitung sowohl als wässrige, wässrig-alkoholische Zubereitung, als wässriges Gel oder Oleogel vorliegen kann ist es erfindungsgemäß vorteilhaft, wenn die Zubereitung in Form einer Emulsion oder Dispersion vorliegt. Erfindungsgemäß besonders bevorzugt sind dabei O/W-Emulsionen.

Liegt die erfindungsgemäße Zubereitung in Form einer O/W-Emulsion vor, so ist sie erfindungsgemäß bevorzugt dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat, Natriumcetearylsulfat enthält.. Ferner ist es vorteilhaft im Sinne der vorliegenden Erfindung Cetearylalkohol in Kombination mit PEG-40 hydriertes Rizinusöl, Natriumcetearylsulfat und Glycerylstearat. Außerdem ist es erfindungsgemäß vorteilhaft Kaliumcetylphosphat als Emulgator einzusetzen.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein. Zusätzlich können Emulgatoren mit einem HLB-Wert < 8 in den O/W-Emulsionen eingesetzt werden wie z.B. Sorbitanstearat.

Erfindungsgemäß vorteilhafte Ausführungsformen der vorliegenden Erfindung sind dadurch gekennzeichnet, dass die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Camosin, natürliche und/oder synthetische Isoflavonoide, Flavonoide, Kreatin, Kreatinin, ß-Alanin, Tocopherylacetat, Dihydroxyaceton; 8-Hexadecen-1,16-dicarbonsäure, Glycerylglycose, Niacinamid, (2-Hydroxyethyl)harnstoff, Vitamin E bzw. seine Derivate und/oder Licochalcon A enthält.

Ferner ist es erfindungsgemäß vorteilhaft, wenn die Zubereitung ein oder mehrere Diole gewählt aus der Gruppe der Verbindungen 2-Methyl-1,3-propandiol, Pentan-1,2-diol, Hexan-1,2-diol, Heptan-1,2-diol, Octan-1,2-diol, Nonan-1,2-diol, Decan-1,2-diol enthält.

Derartige Diole können erfindungsgemäß vorteilhaft in einer Konzentration von 0,1 bis 20 Gewichts-%, bezogen auf das Gesamtgewicht der Zubereitung, in dieser enthalten sein.

Derartige Diole können erfindungsgemäß bevorzugt in einer Konzentration von 1 bis 10 Gewichts-%, bezogen auf das Gesamtgewicht der Zubereitung, in dieser enthalten sein.

Es ist erfindungsgemäß, wenn die Zubereitung ein oder mehrere Parabene (z.B. Methylparaben, Ethylparaben, Propylparaben, Butylparaben) und/oder Phenoxyethanol enthält.

Dabei ist ein Gesamt-Paraben-Gehalt von 0,05 bis 1 Gewichts-%, bezogen auf das Gesamtgewicht der Zubereitung erfindungsgemäß vorteilhaft.

Die erfindungsgemäßen Zubereitungen können ferner vorteilhaft auch Selbstbräunungssubstanzen enthalten, wie beispielsweise Dihydroxyaceton und/oder Melaninderivate in Konzentrationen von 1 Gew.-% bis zu 10 Gew.-%, bezogen auf das Gesamtgewicht der Zubereitung.

Es ist vorteilhaft im Sinne der vorliegenden Erfindung, wenn die Zubereitung nach Anspruch 2 oder 3 Polyacrylate (Carbomer) und/oder vernetztes Acrylat/C10-30Alkylacrylatpolymer enthält.

Polyacrylate (Carbomer) werden erfindungsgemäß vorteilhaft in einer Konzentration 0,01 von 0,5 bis Gewichts-%, bezogen auf das Gesamtgewicht der Zubereitung eingesetzt.

Acrylat/C₁₀₋₃₀ Alkylacrylatpolymer wird erfindungsgemäß vorteilhaft in einer Konzentration von 0,01 bis 0,5 Gewichts-%, bezogen auf das Gesamtgewicht der Zubereitung eingesetzt.

### Vergleichsversuche.

Mit den folgenden Vergleichsversuchen konnte der erfinderische Effekt gezeigt werden:

| **Bestandteile** | **A** | **B** |
|---|---|---|
| Methylparaben | 0,2 | 0,2 |
| Propylparaben | 0,1 | 0,1 |
| Tocopheryl Acetat | 0,1 | 0,1 |
| Cetyl Alcohol | 2,2 | 2,2 |
| Glyceryl Stearat, selbstemulgierend | 0,65 | 0,65 |
| Natrium EDTA | 1 | 1 |
| Phenoxyethanol | 0,5 | 0,5 |
| Cetearyl Alcohol + PEG-40 Rizinusöl + Natrium Cetearyl Sulfat | 2 | 2 |
| Myristyl Myristat | 1,5 | 1,5 |
| C12-15 Alkyl Benzoat | 9 | 9 |
| Phenylbenzimidazol Sulfonsäure | 3 | 3 |
| Glycerin | 0,9 | 0,9 |
| Natronlauge 45%-ig | 1,02 | 1,26 |
| Alkohol Denat. | 8 | 8 |
| Xanthan Gummi | 0,4 | 0,2 |
| Carbomer | 0,1 | 0,3 |
| Titan Dioxid + Trimethoxycaprylylsilan | 1,5 | |
| Butylen Glycol Dicaprylat/Dicaprat | 7 | 7 |
| Parfüm | 0,4 | 0,4 |
| Diethylamino Hydroxybenzoyl Hexyl Benzoat | 3 | 3 |
| Wasser | ad 100 | ad 100 |
| 2,4,6-Tris-(biphenyl)-1,3,5-triazin | 3 | 3 |
| Decylglycosid | 0,45 | 0,45 |
| **SPF in-vivo** | **26** | **38** |
| Colipa Ratio | 2,4 | 2,6 |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

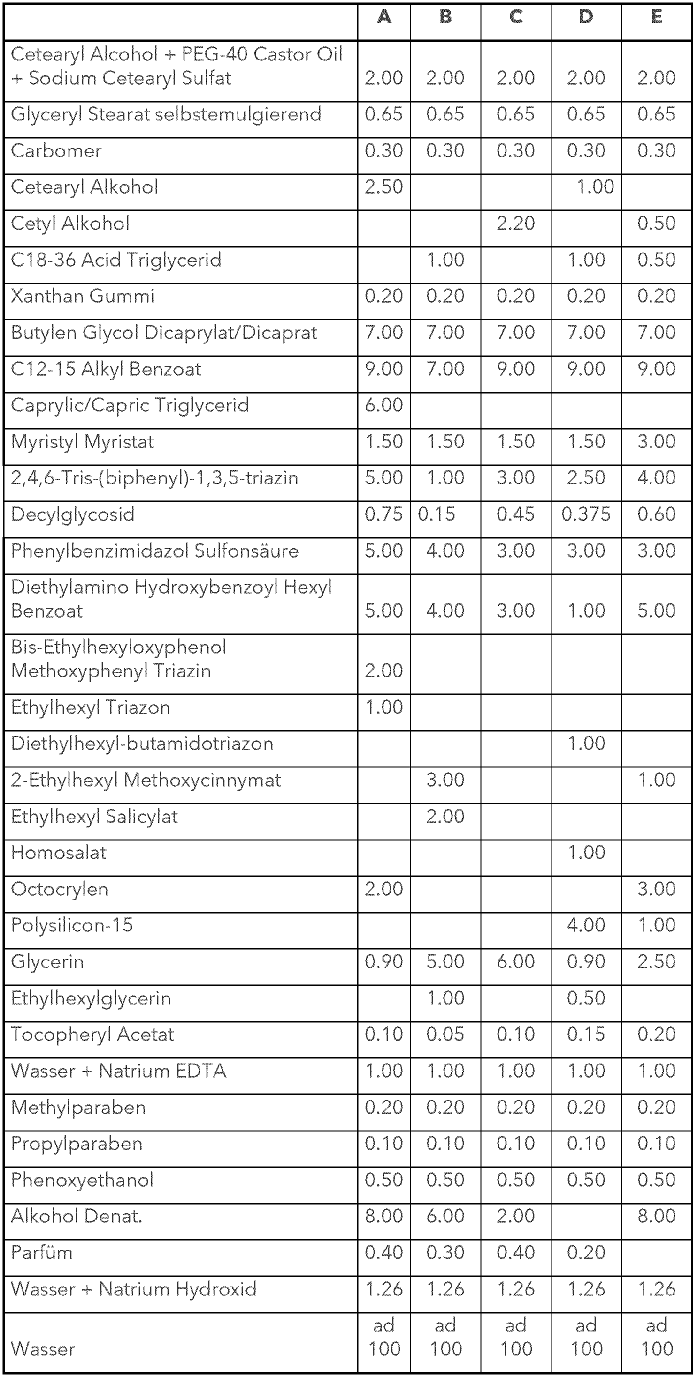

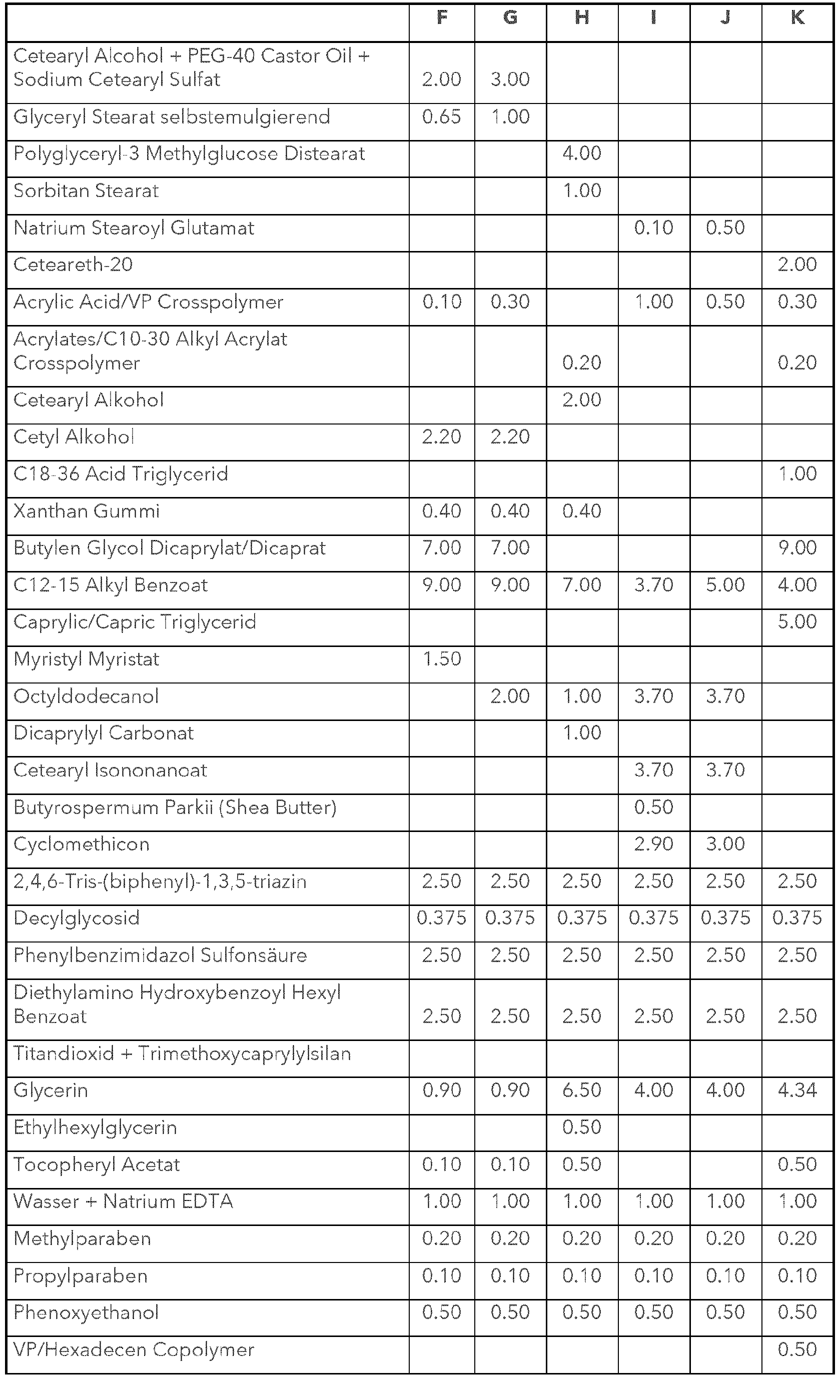

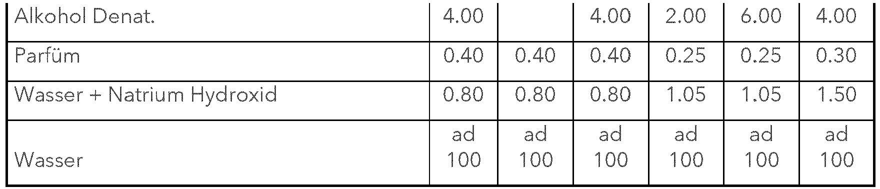

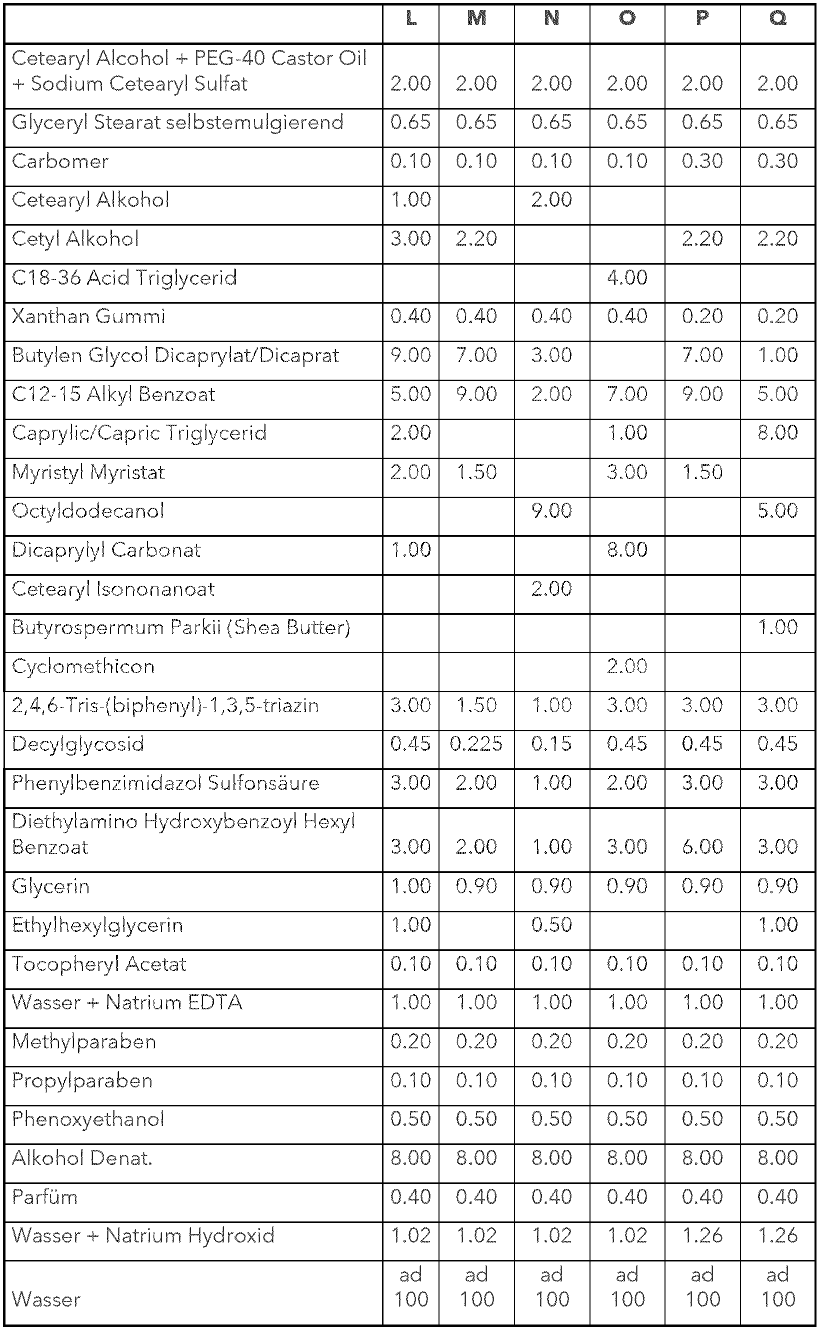

## Patentansprüche

1. Kosmetische Zubereitung enthaltend eine Kombination aus
a) 2,4,6-Tris-(biphenyl)-1,3,5-triazin,
b) Phenylbenzimidazolsulfonsäure und/oder deren Salze,
c) 2-(4'-Diethylamino-2'-hydoxybenzoyl)-benzoesäurehexylester,
wobei die Zubereitung ein oder mehrere Parabene und/oder Phenoxyethanol enthält,
**dadurch gekennzeichnet, dass** die Zubereitung Polyacrylate (Carbomer) enthält.

2. Kosmetische Zubereitung enthaltend eine Kombination aus
a) 2,4,6-Tris-(biphenyl)-1,3,5-triazin,
b) Phenylbenzimidazolsulfonsäure und/oder deren Salze,
c) 2-(4'-Diethylamino-2'-hydoxybenzoyl)-benzoesäurehexylester,
wobei die Zubereitung ein oder mehrere Parabene und/oder Phenoxyethanol enthält,
**dadurch gekennzeichnet, dass** die Zubereitung Ethylhexylsalicylat enthält.

3. Kosmetische Zubereitung enthaltend eine Kombination aus
a) 2,4,6-Tris-(biphenyl)-1,3,5-triazin,
b) Phenylbenzimidazolsulfonsäure und/oder deren Salze;
c) 2-(4'-Diethylamino-2'-hydoxybenzoyl)-benzoesäurehexylester,
wobei die Zubereitung ein oder mehrere Parabene und/oder Phenoxyethanol enthält,
**dadurch gekennzeichnet, dass** die Zubereitung C₁₂₋₁₅ Alkylbenzoat enthält.

4. Zubereitung nach Anspruch 1, 2 oder 3, **dadurch gekennzeichnet, dass** die Zubereitung frei ist von Zinkoxid.

5. Zubereitung nach einem der vorhergehenden Ansprüche, **dadurch gekennzeichnet, dass** die Zubereitung frei ist von Titandioxid.

6. Zubereitung nach einem der vorhergehenden Ansprüche, **dadurch gekennzeichnet, dass** die Zubereitung frei ist weiteren UV-Filtern

7. Zubereitung nach einem der vorhergehenden Ansprüche, **dadurch gekennzeichnet, dass** die Zubereitung in Form einer Öl-in-Wasser Emulsion vorliegt.

8. Kosmetische Zubereitung nach einem der vorhergehenden Ansprüche 1-5 und 7, **dadurch gekennzeichnet, dass** die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze ; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2-(2H-benzotriazol-2-yl)-4-methyl-6-[2-methyl-3-[1,3,3,3-tetramethyl-1-[(trimethylsilyl)oxy]disiloxanyl]propyl]-phenol ; 3-(4-Methylbenzyliden)campher ; 3-Benzylidencampher; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon , 2-Hydroxy-4-methoxy-4'-methylbenzophenon; 2,2'-Dihydroxy-4-methoxybenzophenon; 4-(tert.-Butyl)-4'-methoxydibenzoylmethan ; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone ; 2,4-bis-[5-1(dimethylpropyl)benzoxazol-2-yl-(4-phenyl)-imino]-6-(2-ethylhexyl)-imino-1,3,5-triazin) mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,2'-Methylen-bis-(6-(2H-benzotriazol-2-yl)-4-(1,1,3,3-tetramethylbutyl)-phenol); Polysilicone-15; 2,4-Bis-(4'-Di-neopentylaminobenzalmalonat)-6-(4"-butylaminobenzoat)-s-triazin; Merocyanine.

9. Kosmetische Zubereitung nach einem der vorhergehenden Ansprüche, **dadurch gekennzeichnet, dass** die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, ß-Alanin, Tocopherylacetat, Dihydroxyaceton; 8-Hexadecen-1,16-dicarbonsäure, Glycerylglycose, Niacinamid, (2-Hydroxyethyl)harnstoff, Vitamin E bzw. seine Derivate und/oder Licochalcon A enthält.

10. Kosmetische Zubereitung nach einem der vorhergehenden Ansprüche, **dadurch gekennzeichnet, dass** die Zubereitung ein oder mehrere Diole gewählt aus der Gruppe der Verbindungen 2-Methyl-1,3-propandiol, Pentan-1,2-diol, Hexan-1,2-diol, Heptan-1,2-diol, Octan-1,2-diol, Nonan-1,2-diol, Decan-1,2-diol enthält.

## Claims

1. Cosmetic preparation comprising a combination of
a) 2,4,6-tris(biphenyl)-1,3,5-triazine,
b) phenylbenzimidazolesulfonic acid and/or salts thereof,
c) hexyl 2-(4'-diethylamino-2'-hydroxybenzoyl)benzoate,
wherein the preparation comprises one or more parabens and/or phenoxyethanol,
**characterized in that** the preparation comprises polyacrylates (carbomer).

2. Cosmetic preparation comprising a combination of
a) 2,4,6-tris(biphenyl)-1,3,5-triazine,
b) phenylbenzimidazolesulfonic acid and/or salts thereof,
c) hexyl 2-(4'-diethylamino-2'-hydroxybenzoyl)benzoate,
wherein the preparation comprises one or more parabens and/or phenoxyethanol,
**characterized in that** the preparation comprises ethylhexyl salicylate.

3. Cosmetic preparation comprising a combination of
a) 2,4,6-tris(biphenyl)-1,3,5-triazine,
b) phenylbenzimidazolesulfonic acid and/or salts thereof,
c) hexyl 2-(4'-diethylamino-2'-hydroxybenzoyl)benzoate,
wherein the preparation comprises one or more parabens and/or phenoxyethanol,
**characterized in that** the preparation comprises C12-15 alkyl benzoate.

4. Preparation according to Claim 1, 2 or 3, **characterized in that** the preparation is free from zinc oxide.

5. Preparation according to any of the preceding claims, **characterized in that** the preparation is free from titanium dioxide.

6. Preparation according to any of the preceding claims, **characterized in that** the preparation is free from further UV filters.

7. Preparation according to any of the preceding claims, **characterized in that** the preparation is in the form of an oil-in-water emulsion.

8. Cosmetic preparation according to any of preceding Claims 1-5 and 7, **characterized in that** the preparation comprises one or more further UV filters selected from the group of compounds comprising phenylene-1,4-bis(2-benzimidazyl)-3,3'-5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone; 2-hydroxy-4-methoxy-4'-methylbenzophenone; 2,2'-dihydroxy-4-methoxybenzophenone; 4-(tert-butyl)-4'-methoxydibenzoylmethane; homomenthyl salicylate; 2-ethylhexyl 2-hydroxybenzoate; 2-ethylhexyl 2-cyano-3,3-diphenylacrylate; dimethicodiethylbenzalmalonate; 3-(4-(2,2-bis-ethoxycarbonylvinyl)phenoxy)propenyl)methoxysiloxane/dimethylsiloxane copolymer; dioctylbutylamidotriazone (INCI: Diethylhexyl Butamidotriazone; 2,4-bis[5-1(dimethylpropyl)benzoxazol-2-yl-(4-phenyl)imino]-6-(2-ethylhexyl)imino-1,3,5-triazine) with the CAS No. 288254-16-0; tris(2-ethylhexyl) 4,4',4"-(1,3,5-triazine-2,4,6-triyltriimino)trisbenzoate (also: 2,4,6-tris[anilino(p-carbo-2'-ethyl-1'-hexyloxy)]-1,3,5-triazine, (INCI: Ethylhexyl Triazone); 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); 2,2'-methylenebis(6-(2H-benzotriazol-2-yl)-4-(1,1,3,3-tetramethylbutyl)phenol); polysilicone-15; 2,4-bis(4'-dineopentylaminobenzalmalonate)-6-(4"-butylaminobenzoate)-s-triazine; merocyanines.

9. Cosmetic preparation according to any of the preceding claims, **characterized in that** the preparation comprises, as further ingredients, one or more compounds selected from the group of compounds comprising alpha-lipoic acid, folic acid, phytoene, D-biotin, coenzyme Q10, alpha-glucosylrutin, carnitine, carnosine, natural and/or synthetic isoflavonoids, flavonoids, creatine, creatinine, β-alanine, tocopheryl acetate, dihydroxyacetone; 8-hexadecene-1,16-dicarboxylic acid, glycerylglycose, niacinamide, (2-hydroxyethyl)urea, vitamin E and its derivatives and/or licochalcone A.

10. Cosmetic preparation according to any of the preceding claims, **characterized in that** the preparation comprises one or more diols selected from the group of compounds comprising 2-methyl-1,3-propanediol, pentane-1,2-diol, hexane-1,2-diol, heptane-1,2-diol, octane-1,2-diol, nonane-1,2-diol, decane-1,2-diol.

## Revendications

1. Préparation cosmétique contenant une combinaison de
a) 2,4,6-tris-(biphényl)-1,3,5-triazine,
b) acide phénylbenzimidazolsulfonique et/ou ses sels,
c) ester hexylique de l'acide 2-(4'-diéthylamino-2'-hydoxybenzoyl)-benzoïque,
la préparation contenant un ou plusieurs parabènes et/ou du phénoxyéthanol,
**caractérisée en ce que** la préparation contient des polyacrylates (carbomère).

2. Préparation cosmétique contenant une combinaison de
a) 2,4,6-tris-(biphényl)-1,3,5-triazine,
b) acide phénylbenzimidazolsulfonique et/ou ses sels,
c) ester hexylique de l'acide 2-(4'-diéthylamino-2'-hydoxybenzoyl)-benzoïque,
la préparation contenant un ou plusieurs parabènes et/ou du phénoxyéthanol,
**caractérisée en ce que** la préparation contient du salicylate éthylhexyle.

3. Préparation cosmétique contenant une combinaison de
a) 2,4,6-tris-(biphényl)-1,3,5-triazine,
b) acide phénylbenzimidazolsulfonique et/ou ses sels,
c) ester hexylique de l'acide 2-(4'-diéthylamino-2'-hydoxybenzoyl)-benzoïque,
la préparation contenant un ou plusieurs parabènes et/ou du phénoxyéthanol,
**caractérisée en ce que** la préparation contient un benzoate d'alkyle en C₁₂₋₁₅.

4. Préparation selon la revendication 1, 2 ou 3 **caractérisée en ce que** la préparation est exempte d'oxyde de zinc.

5. Préparation selon l'une quelconque des revendications précédentes, **caractérisée en ce que** la préparation est exempte de dioxyde de titane.

6. Préparation selon l'une quelconque des revendications précédentes, **caractérisée en ce que** la préparation est exempte d'autres filtres des UV.

7. Préparation selon l'une quelconque des revendications précédentes, **caractérisée en ce que** la préparation se trouve sous la forme d'une émulsion huile-dans-eau.

8. Préparation cosmétique selon l'une quelconque des revendications 1 à 5 et 7, **caractérisée en ce que** la préparation contient un ou plusieurs autres filtres des UV, choisis dans le groupe des composés sels de l'acide phénylène-1,4-bis-(2-benzimidazyl)-3,3'-5,5'-tétrasulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidènecamphre ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; 4-(tert-butyl)-4'-méthoxydibenzoylméthane ; salicylate d'homomenthyle ; 2-hydroxybenzoate de 2-éthylhexyle ; 2-cyano-3,3-diphénylacrylate de 2-éthylhexyle ; benzalmalonate de diméthicodiéthyle ; copolymère de 3-(4-(2,2-bis-éthoxycarbonylvinyl)-phénoxy)propényl)-méthoxysiloxane / diméthylsiloxane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine) présentant le n° CAS 288254-16-0 ; ester tris(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone)) ; 2,4-bis-{[4-(2-éthylhexyloxy)-2-hydroxy]-phényl}-6-(4-méthoxyphényl)-1,3,5-triazine (INCI: Bis-Ethylhexyloxyphenol Methoxyphenyl Triazin) ; 2,2'-méthylène-bis-(6-(2H-benzotriazol-2-yl)-4-(1,1,3,3-tétraméthylbutyl)-phénol) ; Polysilicone-15 ; 2,4-bis-(4'-aminobenzalmalonate de di-néopentyle)-6-(4"-aminobenzoate de butyle)-s-triazine ; mérocyanines.

9. Préparation cosmétique selon l'une quelconque des revendications précédentes, **caractérisée en ce que** la préparation contient comme autres constituants un ou plusieurs composés choisis dans le groupe des composés acide alpha-lipoïque, acide folique, phytoène, D-biotine, coenzyme Q10, alpha-glucosylrutine, carnitine, carnosine, isoflavonoïdes naturels et/ou synthétiques, flavonoïdes, créatine, créatinine, β-alanine, acétate de tocophéryle, dihydroxyacétone ; acide 8-hexadécène-1,16-dicarboxylique, glycérylglycose, niacinamide, (2-hydroxyéthyl)urée, vitamine E ou, selon le cas, ses dérivés et/ou licochalcone A.

10. Préparation cosmétique selon l'une quelconque des revendications précédentes, **caractérisée en ce que** la préparation contient un ou plusieurs diols choisis dans le groupe des composés 2-méthyl-1,3-propanediol, pentane-1,2-diol, hexane-1,2-diol, heptane-1,2-diol, octane-1,2-diol, nonane-1,2-diol, décane-1,2-diol.
